# EUROPÄISCHE PATENTSCHRIFT

(11) **EP 2 859 860 B1**
(45) Veröffentlichungstag und Bekanntmachung des Hinweises auf die Patenterteilung: **28.06.2017**
(21) Anmeldenummer: 13187651.8
(22) Anmeldetag: 08.10.2013
(51) Int. Cl.: A61B 18/08, A61B 18/14, A61B 18/00, A61B 18/04, A61B 17/00, A61B 17/3203

(54) **Multifunktionsinstrument**
Multifunctional instrument
Instrument multifonction

(43) Veröffentlichungstag der Anmeldung: 15.04.2015
(73) Patentinhaber: Erbe Elektromedizin GmbH, 72072 Tübingen (DE)
(72) Erfinder: Dürr, Melanie, 72145 Hirrlingen (DE); Beller, Jürgen, 72810 Gomaringen (DE); Selig, Peter, 72379 Hechingen (DE)
(74) Vertreter: Bohnenberger, Johannes

(56) Entgegenhaltungen:
- EP-A1- 2 641 556
- US-A1- 2007 112 342
- US-A1- 2009 125 009
- US-A1- 2011 022 041
- US-A1- 2011 224 667
- US-A1- 2012 265 190
- US-A1- 2013 030 545
- US-B1- 6 270 493

## Beschreibung

Die Erfindung betrifft ein Multifunktionsinstrument zur HF-chirurgischen Behandlung von Gewebe gemäß Anspruch 1.

Instrumente zur HF-chirurgischen Behandlung von Gewebe sind grundsätzlich bekannt. Problematisch sind hierbei häufig unerwünschte Nebeneffekte, die durch den Stromfluss durch das Gewebe des Patienten, insbesondere im jeweiligen Operationsgebiet resultieren können. Diese unerwünschten Nebeneffekte treten insbesondere bei großflächigen und oberflächlichen Verfahren wie der Argon Plasma Koagulation (APC) oder der Spray-Koagulation auf. Durch die dort verwendeten hohen Spannungen, die bis zu 4500 V betragen, können bei elektronischen Peripheriegeräten im Operationsumfeld Störungen durch elektromagnetische Felder hervorgerufen werden. Diese können u. a. negative Auswirkungen auf die Patientenüberwachung, beispielsweise auf das EKG haben. Bei minimal invasiven Eingriffen ist außerdem der Einsatz einer endoskopischen oder laparoskopischen Kamera erforderlich, deren Bild durch die hohe Spannung und die dadurch erzeugten elektromagnetischen Felder negativ beeinflusst werden kann. Um entsprechende Interferenzen zu vermeiden, müssen daher entsprechend hoch isolierende Werkstoffe zur Abschirmung der entsprechenden Geräte eingesetzt werden.

Ein weiterer Nachteil herkömmlicher HF-chirurgischer Verfahren sind Karbonisationseffekte des behandelten Gewebes sowie neuromuskuläre Stimulationen, die durch den HF-Strom induziert werden. Schließlich ist ein weiterer Nachteil von herkömmlichen HF-chirurgischen Instrumenten die Notwendigkeit, für unterschiedliche HF-chirurgische Behandlungsschritte wie das Schneiden, Unterspritzen oder Koagulieren von Gewebe, einen Instrumentenwechsel vorzunehmen, was den Operationsablauf behindert. Weiterhin soll eine thermische Gewebebehandlung mittels HF-chirurgischer Koagulation so gewebeschonend wie möglich durchgeführt werden. Hierzu gehört auch eine Verringerung der Koagulationstiefe bei gleichzeitiger Vergrößerung der flächigen Ausdehnung des Koagulationsgebiets.

Aus der US-A1-2012/265190 ist eine Ablationssonde bekannt, bei der eine erwärmte Flüssigkeit über Seitenöffnungen ausgebracht wird. Die erwärmte Flüssigkeit soll dazu dienen, den Energie-, insbesondere den Wärmeeintrag, in das Gewebe zu erhöhen. Die Ablationssonde hat eine HF-Elektrode.

Die US-A1-2013/030545 beschreibt ein Werkzeug zur Einbringung eines Implantats. Das Werkzeug dient dazu, ein Loch zur Einbringung des Gewebes zu erzeugen. Gemäß dem Dokument gibt es zahlreiche Variationen, wie das Werkzeug konkret ausgestaltet sein kann. Unter anderem wird das Vorsehen eines Bohrers, eines Ultraschallkopfs und eines Wasserstrahlapplikators beschrieben.

Aufgabe der vorliegenden Erfindung ist es daher, ein Multifunktionsinstrument zu schaffen, das für verschiedene Behandlungsschritte keinen Instrumentenwechsel erfordert, und darüber hinaus Nebeneffekte wie Interferenzen mit Peripheriegeräten, Karbonisationseffekte und neuromuskuläre Stimulationen vermieden werden.

Zur Lösung der oben genannten Aufgabe wird ein Multifunktionsinstrument mit den Merkmalen des Anspruchs 1 vorgeschlagen. Das Multifunktionsinstrument gemäß der vorliegenden Erfindung dient u. a. zur HF-chirurgischen Behandlung von Gewebe, insbesondere zum wahlweisen Schneiden, Unterspritzen und Koagulieren von Gewebe. Gemäß der Erfindung weist das Multifunktionsinstrument die folgenden Elemente auf: mindestens eine Fluidkammer zur Aufnahme eines Fluids; eine mit der mindestens einen Fluidkammer in Verbindung stehende Temperierungseinrichtung zur Erwärmung, Erhitzung oder Verdampfung eines in der mindestens einen Fluidkammer vorhandenen Fluids; eine Temperatursteuerung zur Steuerung der Temperatur des in der mindestens einen Fluidkammer vorhandenen Fluids, und wenigstens eine an einem distalen Ende des Multifunktionsinstruments angeordnete und mit der mindestens einen Fluidkammer in Verbindung stehende Fluidaustrittsöffnung.

Ein wesentlicher Punkt der Erfindung liegt somit darin, dass das vorliegende Multifunktionsinstrument ein Fluid nicht nur zum Schneiden und Unterspritzen von Gewebe mit einem zielgerichteten Wasserstrahl einsetzen kann, sondern dass das Fluid darüber hinaus erwärmt, insbesondere erhitzt und sogar verdampft werden kann. Somit kann mittels des Dampfes ein oberflächlicher, thermischer Effekt mit geringer Eindringtiefe während der Koagulation erzeugt und das behandelte Gewebe großflächig devitalisiert werden. Karbonisationseffekte werden hierdurch minimiert. Ferner ist es dadurch möglich, eine Koagulation des Gewebes mit einer niedrigeren Spannung durchzuführen, so dass Interferenzen mit im Operationssaal vorhandenen Peripheriegeräten vermieden werden. Anders als bei herkömmlichen APC- oder Spray-Koagulationsverfahren ist durch den erzeugten Dampf die Koagulationstiefe niedriger und die flächige Ausdehnung des Koagulationseffekts höher. Durch die in das Instrument integrierte Temperierungseinrichtung und die damit verbundene Temperatursteuerung kann das Fluid in der Fluidkammer unmittelbar am Ort der Operation erwärmt, erhitzt oder verdampft werden und somit je nach Bedarf an den jeweiligen Anwendungsfall flexibel angepasst werden.

Besonders vorteilhaft ist es, wenn die Temperierungseinrichtung wenigstens zwei in einem Abstand zueinander angeordnete Temperierungselektroden umfasst, die zumindest bereichsweise eine Wandung der Fluidkammer bilden. Hierbei kann vorgesehen sein, dass die paarweise angeordneten Temperierungselektroden einen radialen Abstand zueinander aufweisen, der insbesondere durch mindestens einen vorzugsweise ringförmig ausgebildeten Abstandshalter festgelegt ist. Mit anderen Worten sind die wenigstens zwei Temperierungselektroden vorzugsweise in axialer Richtung auf derselben Höhe angeordnet, weisen in der radialen Richtung jedoch einen Abstand zur Isolation auf. Auf die Abstandshalter kann verzichtet werden, wenn die Temperierungselektroden an einer Innenfläche einer insbesondere zylindrischen und flexiblen Ummantelung fest angebracht sind und vorzugsweise in Form einer Beschichtung, Folie oder einer sonstigen dünnen Materialschicht ausgebildet sind.

Bei dieser Ausführungsform können die Temperierungselektroden somit ebenfalls flexibel an einer Ummantelung angebracht sein, so dass die Flexibilität der Ummantelung, insbesondere für den Einsatz des Multifunktionsinstruments als laparoskopisches oder endoskopisches Instrument nicht behindert wird. Weiterhin kann vorgesehen sein, dass mehrere Temperierungselektrodenpaare in der axialen Richtung der Ummantelung des Multifunktionsinstruments vorgesehen sind und insbesondere segmentartig an der Innenfläche der Ummantelung angebracht sind. Auf diese Weise wird vermieden, dass eine durchgängige Elektrodenfläche in der axialen Richtung der Ummantelung bei einer Biegung der Ummantelung bricht und somit ein Stromfluss unterbrochen wird.

Zur Steuerung der Temperatur des in der Fluidkammer befindlichen Fluids kann die Temperatursteuerung die der Temperierungseinrichtung, insbesondere den Temperierungselektroden zugeführte elektrische Leistung in Abhängigkeit von einer vorgegebenen Fluid-Durchflussmenge einstellen. Auf diese Weise ist es möglich, die Temperatur des Fluids ohne zusätzliche Sensoren durch die dem HF-Chirurgiegerät bekannte Durchflussmenge und die zugeführte elektrische Leistung zu bestimmen. Die Temperaturdifferenz entspricht dabei dem Quotienten aus der zugeführten Energie und der Wärmekapazität gemäß der folgenden Formel: ΔT=ΔE/(c*m).

Besonders bevorzugt wird auch ein Multifunktionsinstrument, bei dem die wenigstens eine Fluidaustrittsöffnung in einer hohlleiterförmig ausgebildeten aktiven Elektrode angeordnet ist, die insbesondere über ein distales Ende einer Ummantelung hinaus ragt. Die hohlleiterförmige aktive Elektrode ist vorzugsweise zylindrisch ausgebildet. Um zu vermeiden, dass bei der Dampferzeugung flüssiges Wasser in die hohlleiterförmige Elektrode eintritt, ragt das proximale Ende der aktiven Elektrode vorzugsweise in die Fluidkammer hinein. Auf diese Weise sammeln sich nicht verdampfte Fluidtropfen im distalen Bereich zwischen der aktiven Elektrode und der Wandung der Fluidkammer, so dass die Flüssigkeit nicht durch die hohlleiterförmige aktive Elektrode austreten kann. Um einen ausreichend großen Sammelraum für nicht verdampftes Fluid am distalen Ende der Fluidkammer zu schaffen, ist der Durchmesser der mindestens einen Fluidkammer vorzugsweise größer als der Durchmesser der aktiven Elektrode.

Sofern das distale Ende der aktiven Elektrode über ein distales Ende einer Ummantelung des Multifunktionsinstruments hinaus ragt, kann die aktive Elektrode in vorteilhafter Weise sowohl als mechanisches Schneidinstrument als auch als Koagulationselektrode verwendet werden. Um die Temperierungselektroden getrennt von der aktiven Elektrode ansteuern zu können, verfügen die beiden Elektrodentypen vorzugsweise über getrennte Anschlüsse zur Zuleitung eines hochfrequenten Stroms von einem oder mehreren HF-Generatoren. Besonders vorteilhaft ist noch, wenn das distale Ende der aktiven Elektrode als Schneidwerkzeug zum mechanischen Schneiden von Gewebe ausgebildet ist. Insbesondere ist hierbei vorgesehen, dass das distale Ende der aktiven Elektrode zum mechanischen Schneiden eine Schneidkante oder der gleichen Schneidinstrument aufweist.

Besonders vorteilhaft ist ein Multifunktionsinstrument gemäß der vorliegenden Erfindung, das nicht nur eine einzige, sondern zwei oder mehr Fluidaustrittsöffnungen aufweist, die an dem distalen Ende des Multifunktionsinstruments angeordnet sind. Die ein oder mehreren Fluidaustrittsöffnungen können dabei mit einer einzigen Fluidkammer oder mit mehreren voneinander getrennten Fluidkammern in Verbindung stehen. Insbesondere kann die wenigstens eine und jede weitere Fluidaustrittsöffnung als düsenartige axiale Durchgangsöffnung in einem mit der Ummantelung am distalen Ende des Instruments verbundenen Instrumentenaufsatz ausgebildet sein. Auf diese Weise kann von dem Multifunktionsinstrument nicht nur ein gerichteter Wasserstrahl sondern gleichzeitig oder alternativ auch Dampf abgegeben werden. Die Positionierung der Durchgangsöffnungen, insbesondere in dem Instrumentenaufsatz am distalen Ende des Multifunktionsinstruments kann symmetrisch oder unsymmetrisch und insbesondere zentrisch oder exzentrisch erfolgen. Beispielsweise kann vorgesehen sein, die aktive Elektrode mit der mindestens einen Fluidaustrittsöffnung zentrisch in dem Instrumentenaufsatz anzuordnen, während eine oder mehrere weitere Fluidaustrittsöffnungen exzentrisch um die aktive Elektroden-Fluidaustrittsöffnung herum angeordnet sind. Bei mehr als zwei Fluidaustrittsöffnungen kann insbesondere eine konzentrische Anordnung um eine zentrische Fluidaustrittsöffnung im Bereich der aktiven Elektrode vorgesehen sein.

Um den Fluidaustritt aus der einen oder den mehreren Fluidaustrittsöffnungen zu steuern, insbesondere in Abhängigkeit von dem in der Fluidkammer herrschenden Fluid- oder Dampfdruck, kann eine geeignete Ventileinrichtung vorgesehen sein. Die Ventileinrichtung kann beispielsweise als passives Kugel- oder als aktives Piezoventil ausgebildet sein. Auf diese Weise lassen sich die Fluidaustritts-öffnungen, die mit einer einzigen Fluidkammer verbunden sind, je nach Anwendungsfall beliebig ansteuern und insbesondere öffnen und schließen, so dass das Multifunktionsinstrument für viele verschiedene Anwendungsfälle zum Einsatz kommen kann. Die Ventileinrichtung ist dabei vorzugsweise an einem proximalen Ende der Fluidaustrittsöffnung angeordnet, insbesondere an einem proximalen Ende des Instrumentenaufsatzes. Alternativ kann vorgesehen sein, dass zwei unterschiedliche und voneinander getrennte Wasserzuführungen und damit zwei voneinander getrennte Fluidkammern vorgesehen sind, deren Fluidfluss unabhängig voneinander gesteuert wird, wobei jeder Fluidkammer eine separate Fluidaustrittsöffnung zugeordnet ist. In diesem Fall kann beispielsweise eine Fluidkammer für die Zuführung von einem fokussierten Wasserstrahl zum Wasserstrahlschneiden vorgesehen sein, während eine weitere Fluidkammer zur Zuführung von Flüssigkeit zur Dampferzeugung im Zuge eines Koagulationsvorgangs dient.

Zur Lösung der oben genannten Aufgabe wird auch ein HF-Chirurgiesystem mit den Merkmalen des Anspruchs 15 vorgeschlagen. Das Chirurgiesystem weist einen HF-Generator zur Erzeugung eines hochfrequenten Behandlungsstroms sowie zur Erzeugung eines Temperierungsstroms auf, und umfasst außerdem ein Multifunktionsinstrument gemäß der Erfindung.

Die Erfindung wird im Folgenden anhand der Zeichnung näher erläutert. Es zeigen:
- Figur 1: Eine schematische Schnittdarstellung eines Multifunktionsinstruments gemäß der Erfindung;
- Figur 2: Eine Schnittdarstellung des Multifunktionsinstruments entlang der Schnittlinie A gemäß Figur 1;
- Figur 3: Eine Schnittdarstellung des Multifunktionsinstruments entlang der Schnittlinie B gemäß Figur 1;
- Figur 4: Eine Schnittdarstellung des Multifunktionsinstruments entlang der Schnittlinie C gemäß Figur 1, und
- Figur 5: Eine schematische Schnittdarstellung eines Multifunktionsinstruments nach einer weiteren Ausführungsform der Erfindung.

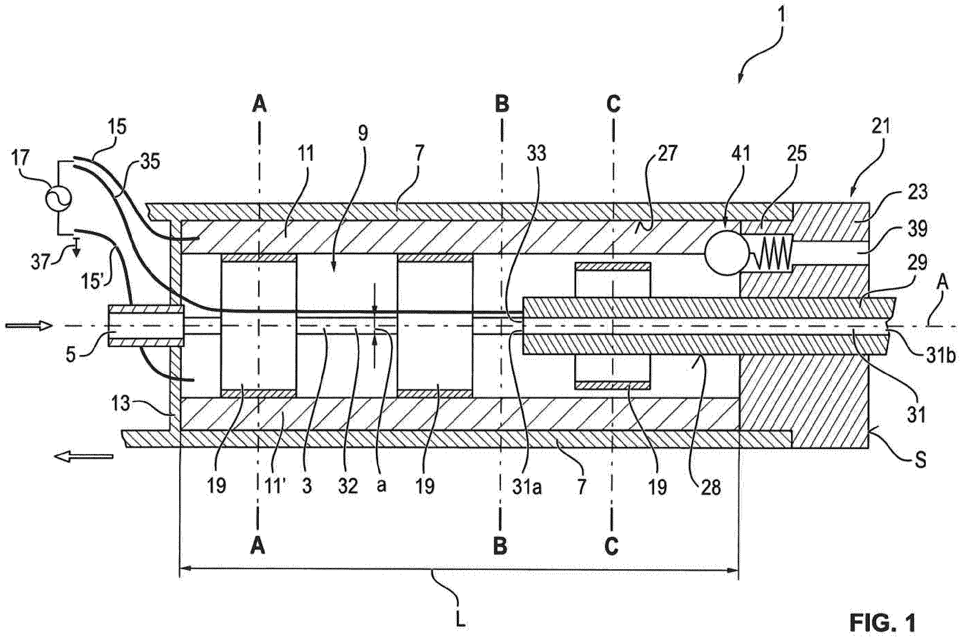

Figur 1 zeigt eine schematische Schnittdarstellung eines Multifunktionsinstruments 1 gemäß der Erfindung. Das Multifunktionsinstrument 1 umfasst eine Fluidkammer 3 zur Aufnahme eines zur Behandlung des menschlichen Körpers geeigneten Fluids, beispielsweise Wasser oder eine physiologische Kochsalzlösung (0,9 % NaCl). Das Fluid gelangt über einen Fluidzulauf 5 in die Fluidkammer 3.

Das Multifunktionsinstrument 1 umfasst weiterhin eine Ummantelung 7, welche die Fluidkammer 3 umgibt. Außerdem ist eine Temperierungseinrichtung 9 in Form von paarweise angeordneten Temperierungselektroden 11, 11' vorgesehen, die zumindest bereichsweise die Wandung der Fluidkammer 3 bilden. In der axialen Richtung A des Multifunktionsinstruments 1 wird die Fluidkammer 3 begrenzt durch eine Wandung 13, die mit dem Fluidzulauf 5 verbunden ist und eine entsprechende Ausnehmung zur Aufnahme desselben aufweist. Der Fluidzulauf 5 ist vorzugsweise zentrisch in der Wandung 13 angeordnet, so dass die Fluidkammer 3 mit dem Fluidzulauf 5 in Fluidverbindung steht. Der Fluidzulauf 5 ist mit seinem proximalen Ende wiederum mit einer entsprechenden Fluidpumpe oder dergleichen Fluidfördereinrichtung eines Chirurgiesystems verbunden.

Die Temperierungselektroden 11 und 11' sind über elektrische Anschlüsse 15 und 15' mit einem HF-Generator 17 verbunden, der einen hochfrequenten Strom zu den Temperierungselektroden 11 und 11' leitet, und der insbesondere Teil eines HF-Chirurgiegerätes ist. Um einen Kurzschluss zwischen den beiden Temperierungselektroden zu vermeiden, sind diese in einem Abstand a zueinander angeordnet. Um den Abstand a zwischen den beiden Elektroden 11 und 11' zu gewährleisten, sind Abstandshalter 19 vorgesehen, die insbesondere in die Elektroden 11 und
11' eingebettet sind und verhindern, dass diese aneinander stoßen. Bei den Abstandshaltern kann es sich beispielsweise um ringförmige Elemente handeln, die aus einem Keramik- oder einem Kunststoffmaterial ausgebildet sind. Wie die Figur 1 zeigt, sind im vorliegenden Ausführungsbeispiel insgesamt drei Abstandshalter 19 vorgesehen, die sich abschnittsweise in der axialen Richtung A segmentartig zwischen den Temperierungselektroden 11 und 11' erstrecken.

Die Temperierungselektroden 11 und 11' sind aus einem leitfähigen Material ausgebildet und erstrecken sich vorzugsweise über die gesamte Länge L der Fluidkammer 3. Am distalen Ende 21 des Multifunktionsinstruments 1 ist ein Instrumentenaufsatz 23 vorgesehen, der insbesondere formschlüssig in das distale Ende der zylindrischen Ummantelung 7 des Multifunktionsinstruments 1 eingreift und dieses dicht abschließt. Eine entsprechende Stufe 25 des Instrumentenaufsatzes liegt hierzu einerseits an der Innenfläche 27 der Ummantelung und andererseits an der Stirnfläche S der Ummantelung 7 an.

Bei der in Figur 1 gezeigten Ausführungsform der Erfindung ragt eine aktive Elektrode 29 in die Fluidkammer 3 hinein, die als rohrförmiges Element ausgebildet ist. Sie erstreckt sich über eine Stirnfläche S des Instrumentenaufsatzes 23 hinaus und ragt mit ihrem proximalen Ende 33 in die Fluidkammer 3 hinein. Die aktive Elektrode 29 ist vorzugsweise in der axialen Richtung A verschieblich in dem Instrumentenaufsatz 23 gelagert, sodass ein distales Ende 31b der aktiven Elektrode 29 mehr oder weniger weit über die Stirnfläche S des Instrumentenaufsatzes 23 hinausragen kann. Die aktive Elektrode 29 weist weiterhin eine als axiale Durchgangsöffnung ausgebildete Fluidaustrittsöffnung 31 auf, die an ihrem proximalen Ende 31a mit der Fluidkammer 3 in Fluidverbindung steht und deren distales Ende 31b als Düse zum Erzeugen eines Wasserstrahls oder zum Abgeben eines Dampfes an ein Operationsgebiet ausgebildet ist. Die (monopolare) aktive Elektrode 29 ist über einen elektrischen Anschluss 35 mit dem HF-Generator 17 verbunden. Der elektrische Anschluss 35 ist dabei getrennt von den elektrischen Anschlüssen 15 und 15' der Temperierungselektroden 11 und 11' angeordnet. Es sei an dieser Stelle darauf hingewiesen, dass über einen weiteren Anschluss 37 der HF-Generator mit einer nicht gezeigten Neutralelektrode verbunden sein kann, die zur Rückführung eines von der monopolaren aktiven Elektrode 29 in einen Patienten eingeleiteten Strom dient.

In der in Figur 1 gezeigten Ausführungsform der Erfindung umfasst das Multifunktionsinstrument 1 eine weitere Fluidaustrittsöffnung 39, die in einer axialen Richtung in dem Instrumentenaufsatz 23 exzentrisch zu der aktiven Elektrode 29 angeordnet ist. An der der Fluidkammer 3 zugewandten Seite weist die Fluidaustrittsöffnung 39 eine Ventileinrichtung 41 auf, die hier beispielhaft als passives Kugelventil abgebildet ist, welches in Abhängigkeit von einem in der Fluidkammer 3 herrschenden Druck die Fluidaustrittsöffnung 39 öffnet oder schließt. Beispielsweise kann die Ventileinrichtung 41 so ausgebildet sein, dass das Ventil bei einem in der Fluidkammer 3 herrschenden niedrigen Druck, insbesondere wenn das dort vorhandene Fluid verdampft wurde, öffnet und bei höheren Drücken, wenn also das Fluid in flüssiger Form vorliegt, schließt.

Sofern an die Temperierungselektroden 11 und 11' ein hochfrequenter Wechselstrom angelegt wird, wird der Strom zwischen den Elektroden 11 und 11' über das in der Fluidkammer 3 befindliche Fluid geleitet, welches zuvor durch den Fluidzulauf 5 in die Fluidkammer 3 mit einer bestimmten Durchflussgeschwindigkeit eingeleitet wurde. Die Temperatur des in der Fluidkammer 3 befindlichen Fluids kann ohne zusätzliche Sensoren durch die voreingestellte Durchflussmenge und die an die Temperierungselektroden zugeführte elektrische Leistung bestimmt werden. Die Temperaturdifferenz entspricht dabei dem Quotienten aus zugeführter Energie und der Wärmekapazität: ΔT=ΔE/(c*m). Auf diese Weise kann das in der Fluidkammer 3 befindliche Fluid erwärmt, erhitzt oder sogar verdampft werden. Je nach Anwendungsfall kann folglich die Temperatur des in der Fluidkammer 3 befindlichen Fluids gesteuert bzw. geregelt werden.

Das Verhältnis der Durchmesser der Fluidaustrittsöffnungen 31 und 39 ist vorzugsweise derart, dass die zentrische in der aktiven Elektrode 29 ausgebildete Fluidaustrittsöffnung 31 vorzugsweise einen kleineren Durchmesser aufweist, als die exzentrische zweite Fluidaustrittsöffnung 39. Während also die erste Fluidaustrittsöffnung 31 beispielsweise einen Durchmesser von ca. 50-500 µm aufweist, kann die zweite Fluidaustrittsöffnung 39 einen entsprechend größeren Durchmesser aufweisen.

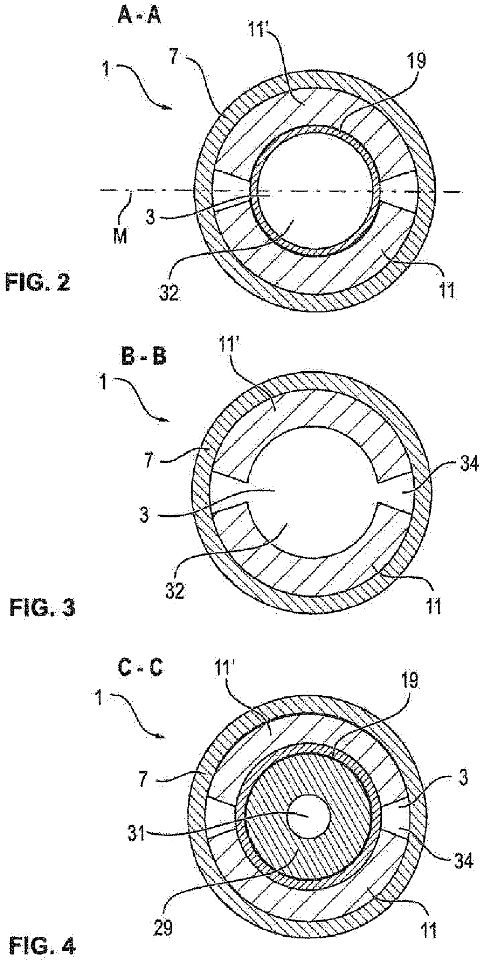

Die Figur 2 zeigt eine Schnittansicht des Multifunktionsinstruments 1 entlang der Schnittlinie A (s. Figur 1). Der Schnitt verläuft durch die Ummantelung 7, die erste Temperierungselektrode 11, den Abstandshalter 19, die Fluidkammer 3 sowie durch die zweite gegenüberliegende Temperierungselektrode 11'. Wie aus der Figur 2 erkennbar ist, ist die Ummantelung 7 des Multifunktionsinstruments 1 im Wesentlichen zylindrisch ausgebildet. Vorzugsweise ist sie aus einem flexiblen Material ausgebildet, so dass das Multifunktionsinstrument als laparoskopisches oder endoskopisches Instrument zur minimalinvasiven Chirurgie eingesetzt werden kann.

Die beiden Temperierungselektroden 11 und 11' sind identisch ausgebildet und im Wesentlichen spiegelbildlich bzgl. einer Mittelebene M des Multifunktionsinstruments angeordnet. Mit anderen Worten sind sie in der axialen Richtung A auf gleicher Höhe angeordnet, während sie in radialer Richtung um 180° versetzt zueinander angeordnet sind. Beide Temperierungselektroden 11 und 11' sind im Wesentlichen C-förmig bzw. halbschalenförmig ausgebildet, so dass ein kreisförmiger Abstandshalter 19 an beide Innenflächen der Temperierungselektroden 11, 11' formschlüssig angreifen kann. Der Abstandshalter 19 sorgt für die Einhaltung eines Isolationsabstandes zwischen den Temperierungselektroden 11, 11'.

Die Figur 3 zeigt eine Schnittdarstellung entlang der in Figur 1 gezeigten Schnittebene B. Der Schnitt verläuft folglich durch die Ummantelung 7, die beiden Temperierungselektroden 11 und 11' sowie durch die Fluidkammer 3. Die Figur 3 macht deutlich, dass die Fluidkammer 3 sowohl einen konzentrisch von den Temperierungselektroden 11, 11' umgebenen inneren Bereich 32 als auch äußere Bereiche 34 umfasst, die jeweils zwischen zueinander beabstandeten umfangsseitigen Endabschnitten der Temperierungselektroden 11, 11' gebildet werden. Über einen radial äußeren Bereich 34 kann im Übrigen die exzentrische zweite Fluidaustrittsöffnung 39 mit der Fluidkammer 3 in Verbindung stehen.

Die Figur 4 zeigt noch eine Schnittdarstellung entlang einer Schnittebene C durch das Multifunktionsinstrument 1 (s. Figur 1). Die Schnittebene C erstreckt sich dabei durch die Ummantelung 7, die beiden Temperierungselektroden 11 und 11' durch einen Abstandshalter 19 sowie durch die rohrförmige aktive Elektrode 29. Die Figur 4 macht noch deutlich, dass im Bereich der aktiven Elektrode 29 der Abstandshalter 19 einen größeren Durchmesser aufweist als die übrigen Abstandshalter 19. Die Fluidaustrittsöffnung 31 in der aktiven Elektrode 29 weist im Übrigen einen Durchmesser auf, der im Wesentlichen übereinstimmt mit dem Innendurchmesser des inneren Bereichs 32, der einen inneren zylindrischen Fluidkammerkanal bildet, und der sich mit Hilfe der Abstandshalter 19 zwischen den beiden Temperierungselektroden 11 und 11' ausbildet (s. auch Figur 1).

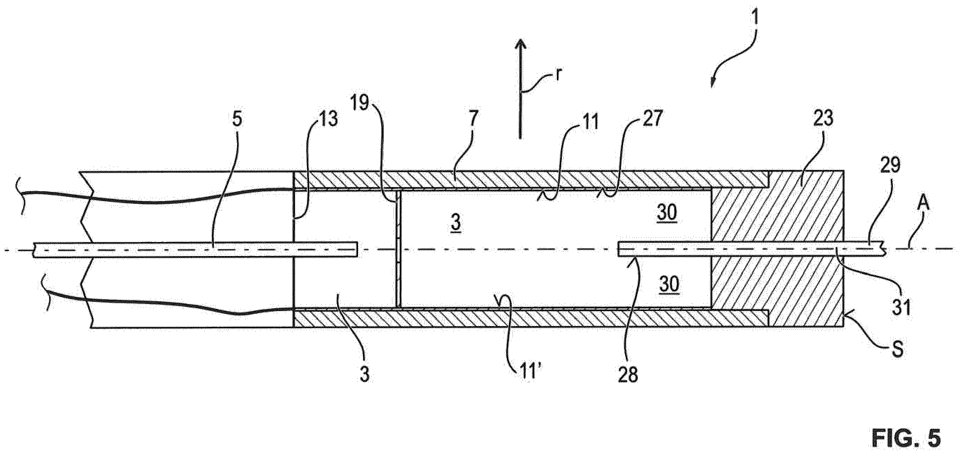

Figur 5 zeigt noch eine weitere Ausführungsform der Erfindung, bei der das Multifunktionsinstrument 1 im Wesentlichen flächige Temperierungselektroden 11 und 11' aufweist, die beispielsweise aus Edelstahl ausgebildet sein können. Die Temperierungselektroden 11 und 11' sind in Form einer dünnen Materialschicht auf die Innenfläche 27 der Ummantelung 7 aufgebracht. Insbesondere kann vorgesehen sein, dass entlang der axialen Richtung A des Multifunktionsinstruments eine segmentartige Aufteilung der Temperierungselektroden 11 und 11' vorgesehen ist, um ein Brechen der Elektrode im Falle einer Verbiegung der flexiblen Ummantelung 7 zu vermeiden.

Um bei einem Knicken der Ummantelung 7 einen Kurzschluss zwischen den Temperierungselektroden 11 und 11' zu verhindern, kann auch bei dieser Ausführungsform der Erfindung ein Abstandshalter 19, beispielsweise aus einem Keramikmaterial oder dergleichen Isolationsmaterial, zwischen den Elektroden vorgesehen sein. Es versteht sich, dass der Abstandshalter 19 so ausgebildet sein muss, dass er ein aus dem Fluidzulauf 5 in die Fluidkammer 3 eintretendes und zu der Fluidaustrittsöffnung 31 fließendes Fluid nicht blockiert. Auch in dem gezeigten Beispiel kann eine Fluidaustrittsöffnung 31 als zentrische Durchgangsbohrung in einer aktiven Elektrode 29 ausgebildet sein, die über eine Stirnfläche S eines Instrumentenaufsatzes 23 hinausragt.

Anders als bei der in Figur 1 gezeigten Ausführungsform der Erfindung ist der Durchmesser der aktiven Elektrode 29 vorliegend jedoch deutlich kleiner als der Durchmesser der Fluidkammer 3 in einer radialen Richtung r des Multifunktionsinstruments 1. Auf diese Weise wird zwischen der Innenwandung (Innenfläche 27) der Fluidkammer 3 und der Außenwandung 28 der in die Fluidkammer 3 ragenden aktiven Elektrode 29 ein Fluidsammelraum 30 gebildet, der nicht verdampftes Fluid am Eintreten in die Fluidaustrittsöffnung 31 hindern kann.

Insgesamt schafft die vorliegende Erfindung ein vorteilhaftes Multifunktionsinstrument, welches einerseits einen fokussierten Wasserstrahl erzeugen kann, und das gleichzeitig in der Lage ist, das hierbei verwendete Fluid durch einen HF-Strom zu erwärmen. Diese Erwärmung kann bis zur Verdampfung des Fluids erfolgen, wobei der hierzu eingesetzte HF-Strom nicht über den Patienten, sondern ausschließlich über die Anschlüsse 15, 15' innerhalb des Instruments fließt. Es handelt sich somit um einen von dem Patientenstromkreis getrennten Stromkreis, der die Temperierungseinrichtung betreibt. An dieser Stelle sei darauf hingewiesen, dass die Temperierungseinrichtung grundsätzlich auch induktiv betrieben werden kann.

Weiterhin ist durch die vorliegende Erfindung mit einem einzigen Multifunktionsinstrument die mechanische Behandlung oder Unterspritzung von biologischem Gewebe mithilfe eines Wasserstrahls möglich. Hierbei kann gleichzeitig mittels eines erwärmten Fluids ein Koagulationseffekt erzeugt werden, indem an die aktive Elektrode ein HF-Strom angelegt wird. Die Temperatur des Wassers kann dabei in besonders vorteilhafter Weise ohne zusätzliche Sensoren durch die dem HF-Chirurgiegerät bekannte Durchflussmenge
und die an die Temperierungselektroden zugeführte elektrische Leistung bestimmt werden.

Zusätzlich kann durch den in der Fluidkammer 3 erzeugten Dampf ein oberflächlicher, thermischer Effekt mit geringer Eindringtiefe erzeugt werden, um das behandelte Gewebe großflächig zu devitalisieren. Hierzu wird ein in der Fluidkammer 3 durch die Temperierungselektroden 11 und 11' erzeugter Dampf über die Fluidaustrittsöffnung 31 und/oder 39 oder über weitere Fluidaustrittsöffnungen an ein Operationsgebiet zugeführt, wobei gleichzeitig an die aktive Elektrode 29 ein HF-Strom angelegt werden kann. Hierbei werden Spannungen von weit unter 4500 V, insbesondere von kleiner als 1000 V und insbesondere kleiner als 500 V angewendet. Anders als bei einer herkömmlichen APC-Koagulation können auf diese Weise Karbonisationseffekte des Gewebes und neuromuskuläre Stimulationen sowie eine negative Beeinflussung von Peripheriegeräten durch eine angelegte Hochspannung vermieden werden.

Weiterhin kann das Multifunktionsinstrument gemäß der Erfindung mit einer metallischen Spitze im Bereich des distalen Endes der aktiven Elektrode 29 auch als klassisches, HF-chirurgisches Schneide- oder Koagulationsinstrument genutzt werden. Durch die Dampferzeugungseinrichtung im Bereich der Fluidkammer 3 können die Spannungen zum HF-chirurgischen Schneiden oder Koagulieren hierbei mit unter 500 V deutlich geringer sein als bei einer herkömmlichen APC- oder Spray-Koagulation.

Die Erhitzung des in der Fluidkammer 3 vorhandenen Fluids wird durch das Einleiten des hochfrequenten Wechselstroms in die Temperierungselektroden 11, 11' bewerkstelligt. Von dort aus fließt der Wechselstrom weiter in das in der Fluidkammer 3 befindliche leitfähige Fluid, welches die Kammer durchströmt. Aufgrund des elektrischen Widerstands erwärmt sich die Flüssigkeit in der Fluidkammer.

Als Elektrodenwerkstoff kommen alle elektrisch leitfähigen Materialen in Betracht, die über die Verwendungsdauer des Instruments widerstandsfähig gegenüber Salzwasser sind. Die Verwendungsdauer beträgt häufig lediglich wenige Stunden, da es sich typischerweise um Einweg-Instrumente handelt, die nach einmaligem Gebrauch entsorgt werden. Insbesondere eignen sich hierzu Edelstahl, leitfähige Keramiken oder leitfähige Kunststoffe. Die Fluidkammer 3 steht in einer Fluidverbindung mit einer oder mehreren Düsen 39, 31 an ihrem distalen Ende, durch die sowohl ein Fluidstrahl erzeugt als auch Dampf abgegeben werden kann. In Abhängigkeit von der Düsengeometrie und dem Fluiddruck kann der Fluidstrahl unterschiedliche Formen, insbesondere Durchmesser, und Richtungen für unterschiedliche Anwendungen aufweisen. Denkbar ist auch die Anordnung mehrerer getrennter oder auch miteinander verbundener Fluidkammern 3, denen wiederum ein oder mehrere Fluidaustrittsöffnungen zugeordnet sein können.

Vorzugweise ist das gesamte Multifunktionsinstrument zylindrisch aufgebaut, insbesondere also die Ummantelung 7, so dass eine Verwendung als laparoskopisches Instrument mit einem Außendurchmesser von beispielsweise 5 mm bis 10 mm oder als endoskopische Sonde mit einem Außendurchmesser von ca. 3 mm bis 1,5 mm möglich ist. Vorteilhaft ist hierbei jedoch ein mechanisch flexibler Aufbau, damit das erfindungsgemäße Multifunktionsinstrument Biegungen innerhalb eines Endoskops folgen kann. Dies kann dadurch bewerkstelligt werden, dass die leitfähigen Temperierungselektroden aus einem segmentierten oder einem gewebeartigen Werkstoff hergestellt werden, wobei es sich um eine dünne Folie handeln kann oder um eine leitfähige Beschichtung, die an der Innenfläche 27 der Ummantelung 7 angebracht ist.

### Bezugszeichenliste:

- 1: Multifunktionsinstrument
- 3: Fluidkammer
- 5: Fluidzulauf
- 7: Ummantelung
- 9: Temperierungseinrichtung
- 11, 11': Temperierungselektroden
- 13: Wandung
- 15, 15': elektrische Anschlüsse
- 17: HF-Generator
- 19: Abstandshalter
- 21: Distales Ende
- 23: Instrumentenaufsatz
- 25: Stufe
- 27: Innenfläche
- 28: Außenwandung
- 29: Aktive Elektrode
- 30: Fluidsammelraum
- 31: Fluidaustrittsöffnung
- 31a: proximales Ende
- 31b: distales Ende
- 32: innerer Bereich
- 33: proximales Ende
- 34: äußerer Bereich
- 35: elektrischer Anschluss
- 37: Anschluss
- 39: Fluidaustrittsöffnung
- 41: Ventileinrichtung
- A: axiale Richtung
- a: Abstand
- L: Länge
- S: Stirnfläche
- r: radiale Richtung
- M: Mittelebene

## Patentansprüche

1. Multifunktionsinstrument (1) zur HF-chirurgischen Behandlung von Gewebe aufweisend:
- mindestens eine Fluidkammer (3) zur Aufnahme eines Fluids;
- eine mit der mindestens einen Fluidkammer (3) in Verbindung stehende Temperierungseinrichtung (9) zur Erwärmung, Erhitzung oder Verdampfung eines in der Fluidkammer (3) vorhandenen Fluids;
- eine Temperatursteuerung zur Steuerung der Temperatur des in der Fluidkammer (3) vorhandenen Fluids, und
- wenigstens eine an einem distalen Ende des Multifunktionsinstruments angeordnete und mit der mindestens einen Fluidkammer (3) in Verbindung stehende Fluidaustrittsöffnung (31), wobei die wenigstens eine Fluidaustrittsöffnung (31) in einer hohlleiterförmig ausgebildeten aktiven Elektrode (29) angeordnet ist, **dadurch gekennzeichnet, dass**
die Elektrode (29) mit der Fluidaustrittsöffnung (31) über ein distales Ende (21) einer Ummantelung (7) des Multifunktionsinstrument (7) hinausragt, wobei die Fluidaustrittsöffnung (31) an einem proximalen Ende (31a) mit der Fluidkammer (3) in Fluidverbindung steht und an einem distalen Ende (31b) als Düse zum Erzeugen eines Wasserstrahls oder zum Abgeben eines Dampfes ausgebildet ist,
wobei das Multifunktionsinstrument (7) dazu ausgebildet ist, den Wasserstrahl zum Schneiden von Gewebe aus der wenigstens einen Fluidaustrittsöffnung (31) abzugeben.

2. Multifunktionsinstrument nach Anspruch 1,
**dadurch gekennzeichnet, dass**
die Temperierungseinrichtung (9) wenigstens zwei in einem Abstand zueinander angeordnete Temperierungselektroden (11, 11') umfasst, die zumindest bereichsweise eine Wandung der Fluidkammer (3) bilden.

3. Multifunktionsinstrument nach Anspruch 2,
**dadurch gekennzeichnet, dass**
die Temperierungselektroden (11, 11') einen radialen Abstand (a) zueinander aufweisen, der insbesondere durch mindestens einen vorzugsweise ringförmig ausgebildeten Abstandshalter (19) festgelegt ist.

4. Multifunktionsinstrument nach einem der Ansprüche 2 oder 3,
**dadurch gekennzeichnet, dass**
die Temperierungselektroden (11, 11') an einer Innenfläche einer insbesondere zylindrischen und flexiblen Ummantelung (7) angebracht sind und vorzugsweise in Form einer Beschichtung, Folie oder einer sonstigen dünnen Materialschicht ausgebildet sind.

5. Multifunktionsinstrument nach einem der vorhergehenden Ansprüche,
**dadurch gekennzeichnet, dass**
die Temperatursteuerung zur Steuerung/Regelung der Fluidtemperatur die der Temperierungseinrichtung (9), insbesondere den Temperierungselektroden (11, 11') zugeführte elektrische Leistung in Abhängigkeit von einer vorgegebenen Fluid-Durchflussmenge einstellt.

6. Multifunktionsinstrument nach Anspruch 5,
**dadurch gekennzeichnet, dass**
die hohlleiterförmig ausgebildete aktive Elektrode (29) in die mindestens eine Fluidkammer (3) hineinragt, und dass insbesondere der Durchmesser der mindestens einen Fluidkammer (3) größer ist als der Durchmesser der aktiven Elektrode (29).

7. Multifunktionsinstrument nach einem der Ansprüche 5 oder 6,
**dadurch gekennzeichnet, dass**
die aktive Elektrode (29) und die Temperierungselektroden (11, 11') über getrennte Anschlüsse (15, 15', 35) zur Zuleitung eines hochfrequenten Stroms verfügen.

8. Multifunktionsinstrument nach einem der Ansprüche 5 bis 7,
**dadurch gekennzeichnet, dass**
das distale Ende (31b) der aktiven Elektrode (29) als Schneidwerkzeug zum mechanischen Schneiden von Gewebe ausgebildet ist, und insbesondere eine Schneidkante aufweist.

9. Multifunktionsinstrument nach einem der vorhergehenden Ansprüche,
**dadurch gekennzeichnet, dass**
wenigstens eine weitere Fluidaustrittsöffnung (39) an einem distalen Ende (21) des Instruments vorgesehen ist, die mit der mindestens einen Fluidkammer (3) oder mit mindestens einer anderen Fluidkammer in Verbindung steht.

10. Multifunktionsinstrument nach Anspruch 9,
**dadurch gekennzeichnet, dass**
die wenigstens eine weitere Fluidaustrittsöffnung (39) als düsenartige axiale Durchgangsöffnung in einem mit der Ummantelung (7) am distalen Ende des Instruments verbundenen Instrumentenaufsatz (23) ausgebildet ist.

11. Multifunktionsinstrument nach Anspruch 10,
**dadurch gekennzeichnet, dass**
die aktive Elektrode (29) in einer axialen Richtung im Wesentlichen zentrisch in dem Instrumentenaufsatz (23) angeordnet und insbesondere verschiebbar darin gelagert ist.

12. Multifunktionsinstrument nach einem der vorhergehenden Ansprüche,
**dadurch gekennzeichnet, dass**
eine oder mehrere der Fluidaustrittsöffnungen (39) mit einer Ventileinrichtung (41) versehen ist, die insbesondere als passives Kugel- oder als aktives Piezoventil ausgebildet ist.

13. Multifunktionsinstrument nach Anspruch 12,
**dadurch gekennzeichnet, dass**
die Ventileinrichtung (41) an einem proximalen Ende der Fluidaustrittsöffnung (39), insbesondere an einem proximalen Ende des Instrumentenaufsatzes (23) angeordnet ist.

14. HF-Chirurgiesystem, aufweisend mindestens einen HF-Generator (17) zur Erzeugung eines hochfrequenten Behandlungsstroms sowie zur Erzeugung eines Temperierungsstroms, und aufweisend ein Multifunktionsinstrument (1) nach einem der Ansprüche 1 bis 13.

## Claims

1. Multifunctional instrument (1) for HF surgical treatment of tissue, having:
- at least one fluid chamber (3) for receiving a fluid;
- a temperature adjuster (9) which communicates with the at least one fluid chamber (3) in order to warm, heat or evaporate a fluid present in the fluid chamber (3);
- a temperature controller for controlling the temperature of the fluid present in the fluid chamber (3), and
- at least one fluid outlet opening (31) arranged at a distal end of the multifunctional instrument and communicating with the at least one fluid chamber (3), wherein the at least one fluid outlet opening (31) is arranged in an active electrode (29) configured in the form of a hollow conductor,
**characterized in that**
the electrode (29) with the fluid outlet opening (31) protrudes beyond a distal end (21) of a sheath (7) of the multifunctional instrument (7), wherein the fluid outlet opening (31), at a proximal end (31a), is fluidically connected to the fluid chamber (3) and, at a distal end (31b), is configured as a nozzle for generating a water jet or for dispensing a vapour,
wherein the multifunctional instrument (7) is designed to dispense the water jet, for cutting tissue, from the at least one fluid outlet opening (31).

2. Multifunctional instrument according to Claim 1, **characterized in that** the temperature adjuster (9) comprises at least two temperature adjustment electrodes (11, 11') which are arranged at a distance from each other and which, at least in some areas, form a wall of the fluid chamber (3).

3. Multifunctional instrument according to Claim 2, **characterized in that** the temperature adjustment electrodes (11, 11') have a radial distance (a) from each other, which distance (a) is determined in particular by at least one preferably annular spacer (19).

4. Multifunctional instrument according to either of Claims 2 and 3, **characterized in that** the temperature adjustment electrodes (11, 11') are mounted on an inner surface of an in particular cylindrical and flexible sheath (7) and are preferably configured in the form of a coating, film or another thin material layer.

5. Multifunctional instrument according to one of the preceding claims, **characterized in that**, in order to control/regulate the fluid temperature, the temperature controller sets the electrical power supplied to the temperature adjuster (9), in particular to the temperature adjustment electrodes (11, 11'), in accordance with a predefined flow rate of fluid.

6. Multifunctional instrument according to Claim 5, **characterized in that** the active electrode (29) configured in the form of a hollow conductor protrudes into the at least one fluid chamber (3), and **in that** in particular the diameter of the at least one fluid chamber (3) is greater than the diameter of the active electrode (29).

7. Multifunctional instrument according to either of Claims 5 and 6, **characterized in that** the active electrode (29) and the temperature adjustment electrodes (11, 11') have separate connections (15, 15', 35) for supplying a high-frequency current.

8. Multifunctional instrument according to one of Claims Claim 5 to 7, **characterized in that** the distal end (31b) of the active electrode (29) is configured as a cutting tool for mechanically cutting tissue and in particular has a cutting edge.

9. Multifunctional instrument according to one of the preceding claims, **characterized in that** at least one further fluid outlet opening (39) is provided at a distal end (21) of the instrument and communicates with the at least one fluid chamber (3) or with at least one other fluid chamber.

10. Multifunctional instrument according to Claim 9, **characterized in that** the at least one further fluid outlet opening (39) is configured as a nozzle-like axial through-hole in an instrument attachment (23) connected to the sheath (7) at the distal end of the instrument.

11. Multifunctional instrument according to Claim 10, **characterized in that** the active electrode (29), in an axial direction, is arranged substantially centrally in the instrument attachment (23) and in particular is mounted movably therein.

12. Multifunctional instrument according to one of the preceding claims, **characterized in that** one or more of the fluid outlet openings (39) is or are provided with a valve device (41), which is configured in particular as a passive ball valve or as an active piezo valve.

13. Multifunctional instrument according to Claim 12, **characterized in that** the valve device (41) is arranged at a proximal end of the fluid outlet opening (39), in particular at a proximal end of the instrument attachment (23).

14. HF surgical system, having at least one HF generator (17) for generating a high-frequency treatment current and also for generating a temperature adjustment current, and having a multifunctional instrument (1) according to one of Claims 1 to 13.

## Revendications

1. Instrument multifonctions (1) destiné au traitement chirurgical par haute fréquence de tissus, lequel présente :
- au moins une chambre à fluide (3) destinée à la réception d'un fluide ;
- un mécanisme de régulation de la température (9) qui se trouve en liaison avec l'au moins une chambre à fluide (3) et qui est destiné au réchauffement, au chauffage ou à la vaporisation d'un fluide se trouvant dans la chambre à fluide (3) ;
- une commande de la température destinée à la commande de la température du fluide se trouvant dans la chambre à fluide (3) ; et
- au moins un orifice de sortie du fluide (31) qui se trouve en liaison avec l'au moins une chambre à fluide (3) et qui est disposé au niveau d'une extrémité distale de l'instrument multifonctions, dans lequel l'au moins un orifice de sortie du fluide (31) est disposé dans une électrode (29) active, laquelle est conçue en forme de conducteur creux,
**caractérisé en ce que**
l'électrode (29) fait saillie avec l'orifice de sortie du fluide (31) par une extrémité (21) distale d'une gaine (7) de l'instrument multifonctions (7), dans lequel l'orifice de sortie du fluide (31) se trouve en liaison fluidique avec la chambre à fluide (3), au niveau d'une extrémité (31a) proximale, et est conçue
au niveau d'une extrémité (31b) distale sous la forme d'une buse, en vue de la production d'un jet d'eau ou en vue de l'émission d'une vapeur,
dans lequel l'instrument multifonctions (7) est conçu en ce sens qu'il émet le jet d'eau, à partir de l'au moins un orifice de sortie du fluide (31), en vue de la découpe de tissus.

2. Instrument multifonctions selon la revendication 1,
**caractérisé en ce que**
le mécanisme de régulation de la température (9) comprend au moins deux électrodes de régulation de la température (11, 11') qui sont disposées à distance l'une par rapport à l'autre et qui forment, tout au moins par endroit, une paroi de la chambre à fluide (3).

3. Instrument multifonctions selon la revendication 2,
**caractérisé en ce que**
les électrodes de régulation de la température (11, 11') présentent une distance (a) radiale l'une par rapport à l'autre, laquelle est définie en particulier par au moins une entretoise (19) qui est conçue de préférence en forme de bague.

4. Instrument multifonctions selon l'une des revendications 2 ou 3,
**caractérisé en ce que**
les électrodes de régulation de la température (11, 11') sont mises en place au niveau d'une surface intérieure d'une gaine (7), qui est en particulier flexible et cylindrique, et sont conçues de préférence sous la forme d'un revêtement, d'un film ou d'une autre couche mince de matériau.

5. Instrument multifonctions selon l'une des revendications précédentes,
**caractérisé en ce que**
la commande de la température destinée à la commande et à la régulation de la température du fluide permet de régler, en fonction d'un débit de fluide prédéterminé, la puissance électrique délivrée au mécanisme de régulation de la température (9), en particulier aux électrodes de régulation de la température (11, 11').

6. Instrument multifonctions selon la revendication 5,
**caractérisé en ce que**
l'électrode (29) active qui est conçue en forme de conducteur creux fait saillie dans l'au moins une chambre à fluide (3) ; et
**caractérisé en ce que**
en particulier, le diamètre de l'au moins une chambre à fluide (3) est supérieur au diamètre de l'électrode (29) active.

7. Instrument multifonctions selon l'une des revendications 5 ou 6,
**caractérisé en ce que**
l'électrode (29) active et les électrodes de régulation de la température (11, 11') disposent de bornes de connexion (15, 15', 35) séparées en vue de l'acheminement d'un courant à haute fréquence.

8. Instrument multifonctions selon l'une des revendications la revendication 5 à 7,
**caractérisé en ce que**
l'extrémité (31b) distale de l'électrode (29) active est conçue sous la forme d'un outil de coupe, destiné à la découpe mécanique des tissus, et présente en particulier un bord tranchant.

9. Instrument multifonctions selon l'une des revendications précédentes,
**caractérisé en ce que**
au moins un autre orifice de sortie du fluide (39) est prévu au niveau d'une extrémité (21) distale de l'instrument multifonctions, lequel orifice de sortie du fluide (39) se trouve en relation avec l'au moins une chambre à fluide (3) ou avec au moins une autre chambre à fluide.

10. Instrument multifonctions selon la revendication 9,
**caractérisé en ce que**
l'au moins un autre orifice de sortie du fluide (39) est conçu, sous la forme d'un orifice de passage axial et en forme de buse, dans un plateau à instruments (23), lequel est relié à la gaine (7) au niveau de l'extrémité distale de l'instrument multifonctions.

11. Instrument multifonctions selon la revendication 10,
**caractérisé en ce que**
l'électrode (29) active est disposée, pour l'essentiel au centre, dans le plateau à instruments (23), dans une direction axiale, et est positionnée en particulier de manière à pouvoir coulisser.

12. Instrument multifonctions selon l'une des revendications précédentes,
**caractérisé en ce que**
un ou plusieurs des orifices de sortie du fluide (39) est doté d'un mécanisme à soupape (41) qui est conçu en particulier sous la forme d'une vanne sphérique passive ou sous la forme d'une vanne piézo active.

13. Instrument multifonctions selon la revendication 12,
**caractérisé en ce que**
le mécanisme à soupape (41) est disposé au niveau d'une extrémité proximale de l'orifice de sortie du fluide (39), en particulier au niveau d'une extrémité proximale du plateau à instruments (23).

14. Système chirurgical à haute fréquence qui présente au moins un générateur à haute fréquence (17) en vue de la production d'un courant de traitement par haute fréquence, ainsi qu'en vue de la production d'un courant de régulation de la température et qui présente un instrument multifonctions (1) selon l'une des revendications 1 à 13.
